(19) **Europäisches Patentamt**
**European Patent Office**
**Office européen des brevets**

(11) **EP 4 223 246 A1**

(12) **EUROPEAN PATENT APPLICATION**
published in accordance with Art. 153(4) EPC

(43) Date of publication:
09.08.2023 Bulletin 2023/32

(51) International Patent Classification (IPC):
*A61B 34/20* (2016.01)          *A61B 34/10* (2016.01)

(21) Application number: 21874003.3

(22) Date of filing: 27.07.2021

(52) Cooperative Patent Classification (CPC):
**A61B 34/10; A61B 34/20;** A61B 34/25; A61B 34/30;
A61B 2034/105; A61B 2034/2048;
A61B 2034/2051; A61B 2034/2057

(86) International application number:
PCT/CN2021/108603

(87) International publication number:
WO 2022/068341 (07.04.2022 Gazette 2022/14)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: 29.09.2020 CN 202011052618

(71) Applicant: Microport Navibot (Suzhou) Co., Ltd.
Suzhou, Jiangsu 215000 (CN)

(72) Inventors:
• **ZENG, Hao**
**Suzhou, Jiangsu 215000 (CN)**
• **LU, Tianwei**
**Suzhou, Jiangsu 215000 (CN)**
• **SHAO, Hui**
**Suzhou, Jiangsu 215000 (CN)**
• **HE, Chao**
**Suzhou, Jiangsu 215000 (CN)**

(74) Representative: **Patentship
Patentanwaltsgesellschaft mbH
Elsenheimerstraße 65
80687 München (DE)**

(54) **READABLE STORAGE MEDIUM, BONE MODELING REGISTRATION SYSTEM AND ORTHOPEDIC SURGICAL SYSTEM**

(57) The present invention provides a readable storage medium, a bone modeling registration system and an orthopedic surgical system. The readable storage medium stores thereon a program, which, when executed, acquires image information of a bone surface of a target object from a stereoscopic vision scanner; creates a first virtual bone model by 3D reconstruction according to the image information of the bone surface; registers the first virtual bone model to a pre-stored second virtual bone model of the target object, and obtains coordinates of the target object in a navigation image coordinate system based on position and posture information of the stereoscopic vision scanner. With this arrangement, a need to place a fiducial marker on the bone is dispensed with, avoiding secondary damage caused to a patient, reducing contact between surgical instruments with the patient's body and the chance of contamination and facilitating cleaning of the instruments. In addition, the system is simple and allows easy deployment of a registration tool during surgery and a simplified registration process.

Fig. 3

## Description

## TECHNICAL FIELD

The present invention relates to the technical field of robot-assisted surgical systems and, in particular, to a readable storage medium, a bone modeling registration system and an orthopedic surgical system.

## BACKGROUND

In recent years, surgical navigation systems have been increasingly used in surgery, especially in orthopedic surgery. For example, MAKO, Robodoc and other navigation systems for orthopedic surgery all combine a robotic arm with an infrared navigation device to enable a robot to assist a surgeon in performing a surgical procedure integrating intraoperative registration according to the surgeon's preoperative planning. Bone registration is a technique for deriving a coordinate transformation between a first virtual bone model in a navigation system and a physical bone. However, the presently commonly used registration tools and methods suffer from the following deficiencies:

(1) A time consuming and cumbersome process is involved. The traditional registration methods require the placement of a fiducial marker at a surgical site of a bone. The placement must be firm enough to avoid easy loosening of the marker, and otherwise the registration process must be started again from the beginning. In particular, registration of the surgical site requires selecting a number of registration points by touching with a probe. However, as this process is demanding on accuracy, its success rate is very low and repeated attempts may be necessary, making the whole process cumbersome and prolonging the surgical procedure.
(2) Their reliability and fault tolerance are poor. Bone registration accuracy must be sufficiently high because it has a direct impact on the accuracy of the entire system and on the outcomes of the surgical procedure. For traditional registration algorithms, a degree of matching between the physical bone and CT data and the selected number and locations of registration points all have an impact on the registration accuracy. In particular, the bone surface is covered with a layer of soft tissue, and the probe must penetrate through this tissue in order to select suitable registration points. This tends to lead to unsatisfactory accuracy of a registration attempt, necessitating an additional attempt. Further, the highest accuracy is usually obtained at the locations that are touched for the selection of registration points, while the matching accuracy at the other locations are not high enough to ensure sufficient overall accuracy, degrading the reliability of the entire system.
(3) Secondary damage may be caused to patients.

As noted above, the existing registration methods for surgery require placing a fiducial marker on a patient's bone such as femur or tibia. This may cause new injuries to the bone and prolong the patient's recovery. In case of insufficient dexterity of the operator, bone screws may be not fastened in place, possibly necessitating re-placement of the fiducial marker and subjecting the patient to additional surgical risk.

## SUMMARY OF THE INVENTION

It is an object of the present invention to overcome the problems in the conventional bone registration techniques by presenting a readable storage medium, a bone modeling registration system and an orthopedic surgical system.
In order to achieve the above object, according to a first aspect of the present invention, it provides a readable storage medium according to a first aspect of the present invention. The readable storage medium stores a program thereon and, when executed, acquires image information of a bone surface of a target object from a stereoscopic vision scanner, creates a first virtual bone model by 3D reconstruction according to the image information of the bone surface, registers the first virtual bone model to a pre-stored second virtual bone model of the target object, and obtains coordinates of the target object in a navigation image coordinate system based on position and posture information of the stereoscopic vision scanner.
Optionally, the image information of the bone surface may contain data of at least two images captured from different views.
Optionally, the creation of the first virtual bone model by 3D reconstruction according to the image information of the bone surface of the at least two images captured from different views may comprise: obtaining point cloud data of a bone contour by segmenting the image information of the bone surface; and creating the first virtual bone model by 3D reconstruction based on the point cloud data of the bone contour.
Optionally, the obtainment of the coordinates of the target object in the navigation image coordinate system based on the position and posture information of the stereoscopic vision scanner when the program stored on the readable storage medium is executed may comprise: deriving a first coordinate transformation between a coordinate system of the stereoscopic vision scanner and a coordinate system of a fiducial marker based on a predetermined connection relationship of the stereoscopic vision scanner and the fiducial marker; deriving a second coordinate transformation between the coordinate system of the fiducial marker and a coordinate system of a navigation device based on a position of the fiducial marker determined by the navigation device; deriving a third coordinate transformation between the coordinate system of the stereoscopic vision scanner and the navigation image coordinate system based on a result of the registration of the first virtual bone model to the second virtual bone model; and obtaining the coordinates of the target object in the navigation image coordinate system by performing a coordinate transformation based on the first, second and third coordinate transformations.

Optionally, the pre-stored second virtual bone model may be created according to an MRI scan of the bone surface of the target object.

Alternatively, the pre-stored second virtual bone model may be created based on a CT scan of the bone surface of the target object, wherein the registration of the first virtual bone model to the pre-stored second virtual bone model of the target object comprises:

deriving cartilage compensation data by a cartilage compensation algorithm; and
correcting the first virtual bone model based on the cartilage compensation data and registering the corrected first virtual bone model to the second virtual bone model.

Optionally, when the program stored on the readable storage medium is executed, the cartilage compensation algorithm may comprise: detecting an edge of a target site for cartilage removal of the target object using the Canny operator; calculating a gradient variation at the edge; fitting a depth variation in a region not for cartilage removal near the target site based on the gradient variation; deriving the cartilage compensation data by iteration until the cartilage has been entirely treated.

Optionally, when the program stored on the readable storage medium is executed, the cartilage compensation algorithm may comprise: detecting edges of a plurality of target sites for cartilage removal of the target object using the Canny operator; calculating gradient variations at the edges; based on the gradient variations, fitting surrounding regions of the target sites to derive depths of removal; and deriving the cartilage compensation data through gradual extension of the fitted regions until the cartilage has been entirely treated.

Optionally, when any two of the fitted regions expend to interface with each other, a depth of removal at the interface may be determined by fitting based on a gradient variation at a center of the target site that is closer to the interface.

Optionally, when the program stored on the readable storage medium is executed, the cartilage compensation algorithm may comprise deriving the cartilage compensation data from a calculation performed on the first virtual bone model by a neural network that has been trained on a training set.

Optionally, when the program stored on the readable storage medium is executed, the registration of the first virtual bone model to the second virtual bone model may comprise:

roughly registering the first virtual bone model to the second virtual bone model;
removing possible error points using a RANSAC algorithm; and
running an ICP algorithm until a predefined convergence condition is satisfied, thereby obtaining a fitted registration matrix.

Optionally, the program stored on the readable storage medium, when executed, may acquire real-time image information of the bone surface of the target object from a stereoscopic vision scanner having a fixed position and posture,

create a real-time first virtual bone model by 3D reconstruction from the real-time image information of the bone surface;
register the first virtual bone model to the pre-stored second virtual bone model of the target object in real time, and
obtain a real-time coordinate of the target object in the navigation image coordinate system.

In order to achieve the above object, according to a second aspect of the present invention, it provides a bone modeling registration system, which comprises a processor and a stereoscopic vision scanner, the stereoscopic vision scanner is configured to capture image information of a bone surface of a target object and feed the image information of the bone surface to the processor,
the processor is communicatively connected to the stereoscopic vision scanner and is configured to: acquire position and posture information of the stereoscopic vision scanner and the image information of the bone surface of the target object captured by the stereoscopic vision scanner; create a first virtual bone model by 3D reconstruction according to the image information of the bone surface; register the first virtual bone model to a pre-stored second virtual bone model; and obtain coordinates of the target object in a navigation image coordinate system based on the position and posture information of the stereoscopic vision scanner.

Optionally, in the bone modeling registration system, the stereoscopic vision scanner may have a fixed position and posture, which may be determined by calibration.

Optionally, the bone modeling registration system may further comprise a navigation device and a fiducial marker, the fiducial marker connected to the stereoscopic vision scanner, the navigation device adapted to the fiducial marker and configured to acquire real-time coordinate information of the fiducial marker and to transmit the real-time coordinate information to the processor, wherein the processor is communicatively connected to the navigation device and configured to obtain the position and posture information of the stereoscopic vision scanner based on the real-time coordinate information of the fiducial marker fed back from the navigation device.

The above object is also attained by an orthopedic surgical system according to a third aspect of the present invention, which comprises the bone modeling registration system as defined above and at least one of a robotic arm, a surgical cart and a navigation cart.

In summary, the present invention provides a readable storage medium, a bone modeling registration system and an orthopedic surgical system. The readable storage medium stores a program thereon and when executed, acquires image information of a bone surface of a target object from a stereoscopic vision scanner; creates a first virtual bone model by 3D reconstruction according to the image information of the bone surface; registers the first virtual bone model to a pre-stored second virtual bone model of the target object, and obtains coordinates of the target object in a navigation image coordinate system based on position and posture information of the stereoscopic vision scanner.

With this arrangement, the coordinates of the target object in the navigation image coordinate system can be obtained by registering the first virtual bone model reconstructed from the image information of the bone surface captured by the stereoscopic vision scanner to the pre-stored second virtual bone model and performing transformations among different coordinate systems. That is, a preoperative image model can be registered to an intraoperative model of a physical bone. A need to place a fiducial marker on the bone is dispensed with, avoiding secondary damage caused to a patient, reducing contact of surgical instruments with the patient's body and the chance of contamination, and facilitating cleaning of the instruments. In addition, the bone modeling registration system is simple and allows easy deployment of the registration tool during surgery, a simplified registration process, lower surgical complexity and reduced surgical time. Further, compared with the traditional touch-based selection approach, the scanning approach employed by the stereoscopic vision scanner is more accurate, can better reflect the structure of a bone surface and enables higher registration accuracy.

## BRIEF DESCRIPTION OF THE DRAWINGS

Those of ordinary skill in the art would appreciate that the following drawings are presented merely to enable a better understanding of the present invention rather than to limit the scope thereof in any sense. In the drawings:

Fig. 1 schematically illustrates a surgical scenario in which an orthopedic surgical system of the present invention is used;
Fig. 2 is a flowchart of an orthopedic surgical procedure performed using an orthopedic surgical system according to the present invention;
Fig. 3 is a schematic illustration of a bone modeling registration system according to Embodiment 1 of the present invention;

Fig. 4 schematically illustrates a target site for cartilage removal according to Embodiment 1;
Fig. 5 schematically illustrates a scan performed after cartilage removal according to Embodiment 1;
Fig. 6 schematically illustrates a coordinate transformation according to Embodiment 1;
Fig. 7 schematically illustrates target sites for cartilage removal according to Embodiment 2 of the present invention;
Fig. 8 schematically illustrates a scan performed after cartilage removal according to Embodiment 2;
Fig. 9 schematically illustrates target sites for cartilage removal according to a Embodiment 3 of the present invention;
Fig. 10 schematically illustrates a scan performed after cartilage removal according to Embodiment 3; and
Fig. 11 is a schematic illustration of a bone modeling registration system according to Embodiment 4 of the present invention.

In these figures,

1 denotes a surgical cart; 2, a robotic arm; 3, a guide fiducial marker; 4, an osteotomy guide; 5, a surgical instrument; 6, a tracker; 7, a secondary display; 8, a primary display; 9, a navigation cart; 10, a keyboard; 12, a femur; 14, a tibia; 17, a patient; 18, an operator; TI, a coordinate system of a navigation device; T2, a coordinate system of a fiducial marker; T3, a coordinate system of a stereoscopic vision scanner; T4, a navigation image coordinate system; 100, a stereoscopic vision scanner; 200, a navigation device; 300, a fiducial marker; and 400, a target site for cartilage removal.

## DETAILED DESCRIPTION

Objects, advantages and features of the present invention will become more apparent upon reading the following more detailed description of the present invention, which is set forth by way of particular embodiments with reference to the accompanying drawings. Note that the figures are provided in a very simplified form not necessarily drawn to exact scale and for the only purpose of facilitating easy and clear description of the embodiments. In addition, the structures shown in the figures are usually partial representations of their actual counterparts. In particular, as the figures would have different emphases, they are sometimes drawn to different scales.

As used herein, the singular forms "a", "an" and "the" include plural referents, and the term "or" is generally employed in the sense of "and/or", "several" of "at least one", and "at least two" of "two or more than two". Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal" generally refer to an end closer to an operator, and the term "distal" generally refer to an end closer to a subject being operated on. The terms "one end" and "the other end", as well as "proximal end" and "distal end", are generally used to refer to opposing end portions including the opposing endpoints, rather than only to the endpoints. The terms "mounting", "coupling" and "connection" should be interpreted in a broad sense. For example, a connection may be a permanent, detachable or integral connection, or a mechanical or electrical connection, or a direct or indirect connection with one or more intervening media, or an internal communication or interaction between two elements. Furthermore, when an element is referred herein to as being disposed on another element, it is typically only meant that the two elements are connected, coupled, mated or geared to each other either directly or indirectly with the presence of intervening element(s). Such reference should not be interpreted as indicating or implying any relative spatial relationship between the two elements. That is, the referenced element may be positioned inside, outside, above, beneath, beside or in any other spatial relationship to the other element, unless the context clearly specifies. Those of ordinary skill in the art can understand the specific meanings of the above-mentioned terms herein, depending on their context.

The present invention seeks principally to overcome the problems in the conventional bone registration techniques by presenting a readable storage medium, a bone modeling registration system and an orthopedic surgical system.

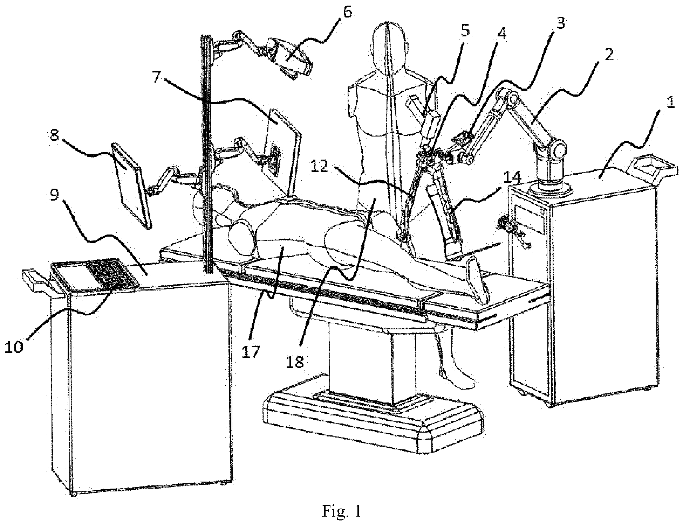

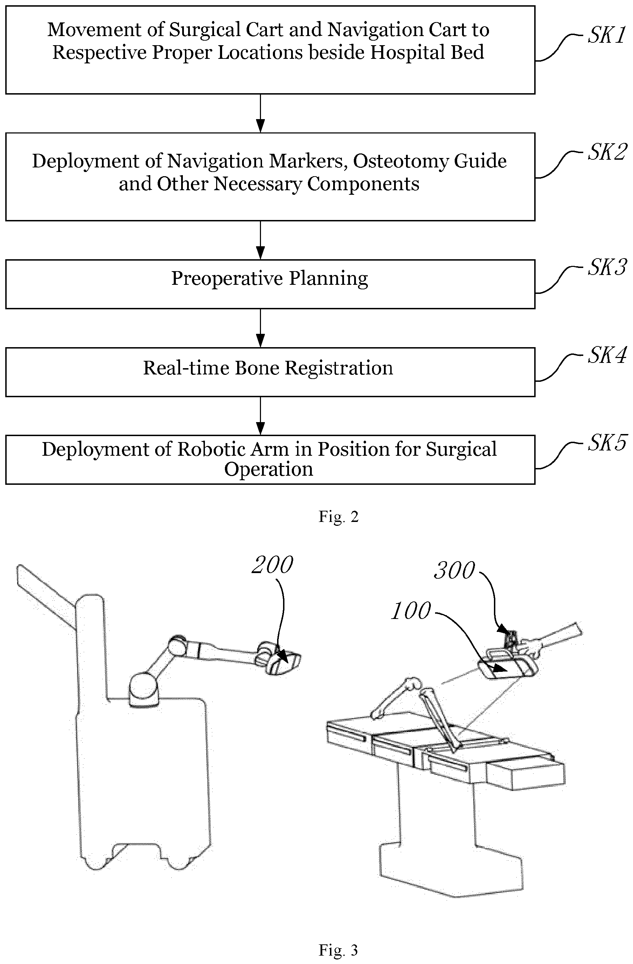

Reference is now made to Figs. 1 and 2. Fig. 1 schematically illustrates a surgical scenario in which an orthopedic surgical system of the present invention is used. Fig. 2 is a flowchart of an orthopedic surgical procedure performed using an orthopedic surgical system according to the present invention.

In the exemplary embodiment shown in Fig. 1, the orthopedic surgical system is used for knee replacement. However, the orthopedic surgical system of the present invention is not limited to being used in any particular environment, as it can also be used in other types of orthopedic surgery. In the following, the orthopedic surgical system will be described in the context of use for knee replacement as an example. However, this shall not be construed as an limitation to the present invention in any sense.

As shown in Fig. 1, the orthopedic surgical system includes a control device, a bone modeling registration system, a robotic arm 2 and an osteotomy guide 4. The robotic arm 2 is disposed on the surgical cart 1. The control device is implemented as a computer in some embodiments, but the present invention is not so limited. The computer is equipped with a processor, a primary display 8 and a keyboard 10. Moreover preferably, it further includes a secondary display 7. The secondary display 7 and the primary display 8 may display the same content, or not. The osteotomy guide 4 is attached to and supported at an end of the robotic arm 2 so as to be adjustable in terms of spatial position and posture.

In some embodiments, the orthopedic surgical system further includes a navigation device, which may be based on electromagnetic, optical or inertial positioning. Preferably, the navigation device is based on optical positioning, which provides higher measurement accuracy and increased positioning accuracy of the osteotomy guide 4, compared to the other navigation techniques. The following description is made in the context of the navigation device being based on optical positioning as an example, but this should not be construed as an limitation in any way.

Optionally, a tracker 6 may be used to capture a signal (preferably, an optical signal) reflected from a fiducial marker 3 on the guide and record a position and posture of the fiducial marker 3 (in a base coordinate system). Subsequently, when a computer program stored in a storage of the control device is executed, the control device may control, based on the current and desired positions and postures of the fiducial marker 3, the robotic arm 2 to move, thus driving the osteotomy guide 4 and the fiducial marker 3 to move, so that the fiducial marker 3 reach the desired position and posture, which is mapped to a desired position and posture of the osteotomy guide 4.

Thus, in applications of the orthopedic surgical system, the osteotomy guide 4 can be automatically positioned. Moreover, during surgery, the fiducial marker 3 tracks and feeds back in real time the position and posture of the osteotomy guide 4, based on which, the robotic arm is controlled to move, resulting in an adjustment in the position and posture of the osteotomy guide 4. In this way, in addition to high positioning accuracy of the osteotomy guide 4 being achievable, the osteotomy guide 4 can be supported on the robotic arm 2 rather than fixed on a patient's body, avoiding causing secondary damage thereto.

In general, the orthopedic surgical system further includes the surgical cart 1 and a navigation cart 9. The control device and part of the navigation device are mounted on the navigation cart 9. For example, the processor may be mounted inside the navigation cart 9, while the keyboard 10 may be placed outside the navigation cart 9 to facilitate manipulation. Additionally, the primary display 8, the secondary display 7 and the tracker 6 may be all fixed to a mast erected upright on a surface of the navigation cart 9, with the robotic arm 2 being mounted on the surgical cart 1. The use of the surgical cart 1 and the navigation cart 9 enables easy surgical operation.

Referring to Fig. 2, the use of the orthopedic surgical system in this embodiment for knee replacement surgery generally involves the steps as follows.

Step SK1: Movement of the surgical cart 1 and the navigation cart 9 to respective proper locations beside a hospital bed.

Step SK2: Deployment of navigation markers, the osteotomy guide 4 and other necessary components (e.g., a sterile bag).

Step SK3: Preoperative planning. Specifically, an operator 18 may accomplish preoperative planning by importing image data from a CT/MRI (Computerized Tomography/Magnetic Resonance Imaging) scan of a bone of a patient 17 to the computer, which then develops an osteotomy plan including, for example, coordinates of a bone surface to be cut, a model of a prosthesis, a target position and posture for the prosthesis and other information. More specifically, based on image data of the patient's knee joint obtained from a CT/MRI scan, the computer may create a virtual three-dimensional (3D) model of the knee joint and develop an osteotomy plan based on the virtual 3D model, which may serve as a basis for a surgical operator to carry out preoperative assessment. Yet more specifically, the osteotomy plan may be developed based on the virtual 3D model of the knee joint, as well as on dimensions of the prosthesis, a target location for an osteotomy plate and the like, and may be output in the form of a surgical report, which may specify a series of reference data including the coordinates of the bone surface to be cut, an amount of bone to be cut away, an osteotomy angle, the dimensions of the prosthesis, the target location for the prosthesis, auxiliary surgical instruments, etc. In particular, it may contain a series of passages of text for the surgical operator's reference, which may specify the reason(s) for the osteotomy angle, for example. The virtual 3D model of the knee joint may be displayed on the primary display 8. During the preoperative planning, the operator may input surgical parameters through the keyboard 10.

Step SK4: Real-time bone registration. Subsequent to the preoperative assessment, positions of feature points of the patient's femur 12 and tibia 14 may be acquired in real time, and the processor may then determine actual positions and postures of the bones using a feature matching algorithm and correlate them to their respective graphic representations. The bone modeling registration system registers the actual positions and postures of the femur 12 and the tibia 14 to the virtual 3D model of the patient's knee joint created in Step SK3 based on the image data from the preoperative CT/MRI scan of the knee joint, thereby mapping the actual positions and postures of the femur 12 and the tibia 14 to coordinates of the robotic arm in a coordinate system of the navigation device. Thus, the robotic arm 2 can perform surgical operation according to the bone positions planned in the navigation device.

Step SK5: Deployment of the robotic arm in position for surgical operation. The navigation device sends the coordinates of the bone surface to be cut that are determined in the preoperative planning to the robotic arm 2, which then locates the bone surface to be cut with the aid of the fiducial marker 3 and moves to a proper location. After causing the robotic arm 2 to remain stationary (i.e., in an holding state), the operator may perform bone cutting and/or drilling operations using a surgical instrument 5 such as a swing saw or an electric drill with the aid of the osteotomy guide 4. Following the completion of the bone cutting and/or drilling operations, the operator may set the prosthesis in place and carry out other necessary operations.

Traditional surgical systems and navigation surgical systems without the participation of a robotic arm in positioning require manual adjustment of an osteotomy guide, which is, however, inaccurate and inefficient. In contrast, by positioning an osteotomy guide with a robotic arm, an operator does not need to fix the osteotomy guide on a bone with additional bone screws, reducing trauma to the patient and surgical time.

Robot-assisted surgery can be achieved based on the above-discussed orthopedic surgical system, which can facilitate an osteotomy procedure by helping an operator identify a target site in need of osteotomy. In order to overcome the problem in the conventional bone registration techniques, the present invention provides a bone modeling registration system including a processor and a stereoscopic vision scanner 100. The processor may be either a common processor inside the computer that is supported on the surgical cart 1, or a standalone processor. The stereoscopic vision scanner 100 is configured to capture image information of a bone surface of a target object in the form of 3D data and feed the image information of the bone surface back to the processor. The processor is communicatively connected to the stereoscopic vision scanner 100 and is configured to acquire position and posture information of the stereoscopic vision scanner 100 and the image information of the bone surface of the target object captured by the stereoscopic vision scanner 100. 3D reconstruction is performed based on the image information of the bone surface to create a first virtual bone model, which is then registered to a pre-stored second virtual bone model. From the position and posture information of the stereoscopic vision scanner 100, coordinates of the target object are derived in a navigation image coordinate system. The orthopedic surgical system of the present invention includes the bone modeling registration system as defined above and is preferred to further include at least one of the robotic arm 2, the surgical cart 1 and the navigation cart 9. With this arrangement, the bone modeling registration system can be used for preoperative or intraoperative bone registration. During configuration, there is no need to place a fiducial marker on the bone, thereby avoiding secondary damage caused to the patient, reducing contact of the human body with surgical instruments, lowering the chance of contamination and facilitating cleaning of the surgical instruments. In addition, the bone modeling registration system is simple and allows easy deployment of the registration tool during surgery, a simplified registration process, lower surgical complexity and reduced surgical time. Further, compared with the traditional touch-based selection approach, the scanning approach employed by the stereoscopic vision scanner is more accurate, can better reflect the structure of a bone surface and enables higher registration accuracy.

The readable storage medium, the bone modeling registration system and the orthopedic surgical system provided in the present invention will be described in detail below with reference to a few embodiments and the accompanying drawings.

**EMBODIMENT 1**

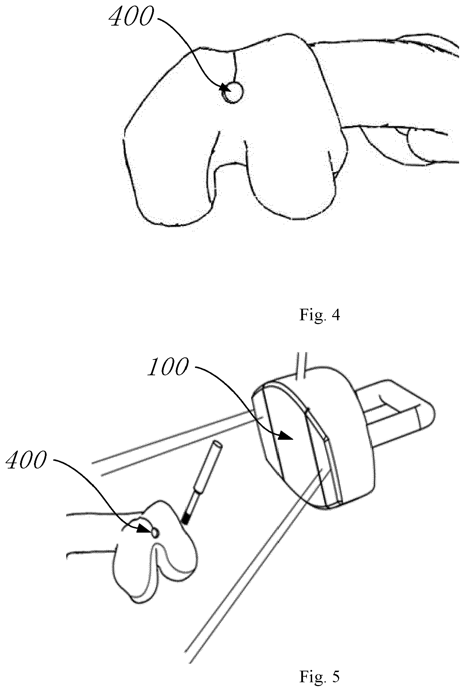

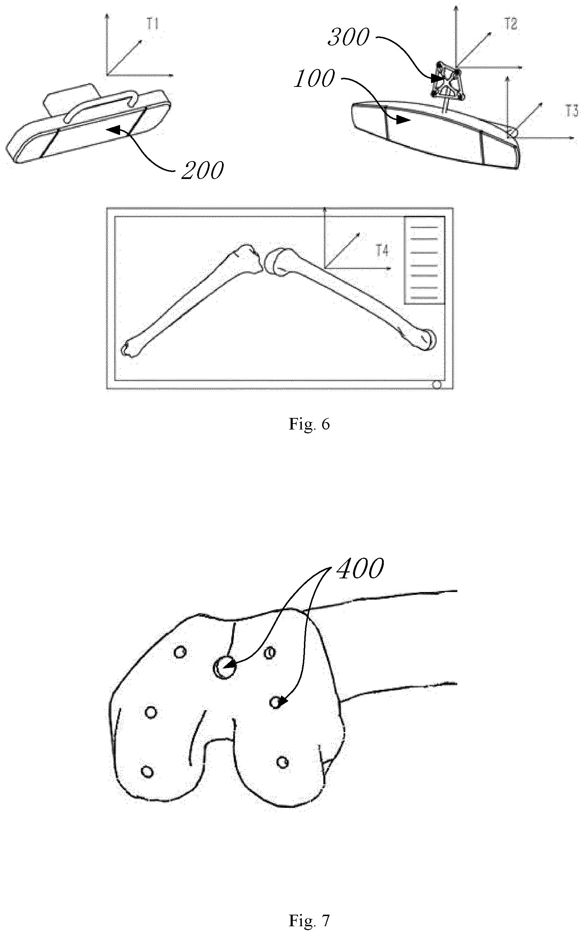

Reference is now made to Figs. 3 to 6. Fig. 3 is a schematic illustration of a bone modeling registration system according to Embodiment 1 of the present invention. Fig. 4 schematically illustrates a target site for cartilage removal according to Embodiment 1 of the present invention. Fig. 5 schematically illustrates a scan performed after cartilage removal according to Embodiment 1 of the present invention. Fig. 6 schematically illustrates a coordinate transformation according to Embodiment 1 of the present invention.

As shown in Fig. 3, the bone modeling registration system of Embodiment 1 includes a processor (not shown) and a stereoscopic vision scanner 100. Preferably, the bone modeling registration system further includes a navigation device 200 and a fiducial marker 300. The navigation device 200 may be implemented either as the aforementioned tracker 6 in the orthopedic surgical system, or as a standalone device. The following description is made in the context of the navigation device 200 being implemented as the tracker 6 as an example, and accordingly, the fiducial marker 300 is an optical fiducial marker adapted to the tracker 6. The fiducial marker 300 is attached to the stereoscopic vision scanner 100, and the navigation device 200 can be adapted to the fiducial marker 300 so as to be able to acquire coordinate information of the fiducial marker 300 in real time and transmit the coordinate information to the processor in real time. The processor is communicatively connected to the navigation device 200 and configured to derive position and posture information of the stereoscopic vision scanner 100 from the real-time coordinate information of the fiducial marker 300 from the navigation device 200.

Specifically, the stereoscopic vision scanner 100 may utilize binocular stereoscopic vision to scan a target object (e.g., a knee joint) and capture accurate 3D depth information over a large range (i.e., 3D data of a surface of the knee joint). Binocular stereoscopic vision is a basis for creating a 3D imaging effect. It is a method based on the principle of parallax, in which an imaging device is used to capture two images of an object from different locations, and 3D geometric information of the object is derived from position deviations between corresponding points in the images. The stereoscopic vision scanner 100 may be selected as an established binocular or trinocular scanner product available on the market.

After receiving the image information (or depth information) of the bone surface from the stereoscopic vision scanner 100, the processor can create a first virtual bone model by 3D reconstruction from the image information of the bone surface. Alternatively, the 3D reconstruction of the image information may be directly accomplished by the stereoscopic vision scanner. The results of the 3D reconstruction are sent to the processor. Preferably, the image information of the bone surface contains data of at least two images captured from different views. Additionally, the processor may acquire a pre-stored second virtual bone model, which may be created from a preoperative CT or MRI scan of the bone surface of the target object.

Optionally, the stereoscopic vision scanner 100 may be secured to the fiducial marker 300 in a known relative positional relationship. In this way, the navigation device 200 can obtain position and posture information of the stereoscopic vision scanner 100 through the fiducial marker 300. Additionally, the navigation device 200 may transmit the position and posture information of the stereoscopic vision scanner 100 to the processor for registration of the first virtual bone model to the pre-stored second virtual bone model. From the position and posture information of the stereoscopic vision scanner 100, coordinates of the target object in a navigation image coordinate system can be derived. In this way, a physical bone can be positionally registered to a navigation image. In some implementations, the stereoscopic vision scanner 100 and the fiducial marker 300 may be in a fixed relative positional relationship, which may be determined, for example, from a mechanical design document or by calibration.

Correspondingly, in Embodiment 1, there is also provided a readable storage medium (e.g., a computer-readable storage medium) storing thereon a program (e.g., a computer program) which, when executed, carries out the steps of:

Step SA1: acquiring image information of a bone surface of a target object from a stereoscopic vision scanner 100;
Step SA2: creating a first virtual bone model by 3D reconstruction from the image information of the bone surface;
Step SA3: registering the first virtual bone model to a pre-stored second virtual bone model; and
Step SA4: deriving coordinates of the target object in a navigation image coordinate system from the stereoscopic vision scanner 100.

Specifically, Step SA2 may further include:

Step SA21: obtaining point cloud data of a bone contour by segmenting the image information of the bone surface; and
Step SA22: creating the first virtual bone model by 3D reconstruction based on the point cloud data of the bone contour. The first virtual bone model may be a reconstructed virtual femur model.

Preferably, Step SA3 includes: roughly registering the first virtual bone model to the second virtual bone model; removing possible error points using a random sample consensus (RANSAC) algorithm; performing an iterative closest point (ICP) algorithm until a predefined convergence condition is satisfied, thereby obtaining a fitted registration matrix. Specifically, point cloud registration is a process for transforming coordinates of a point cloud in a coordinate system to coordinates of the point cloud in a different coordinate system. The algorithm used in Step SA3 is a modified version of the traditional ICP algorithm, which generally includes:

a) determining a corresponding point set $q_i$ in a target point cloud;
b) fitting matrices R and T so that the shortest distance is obtained between a source point cloud $p_i$, and $q_i$, wherein a distance error E is determined according to:

$$E(R,T) = \frac{1}{n} \sum_{i=1}^{n} \| q_i - (R * p_i + T) \|^2$$

c) iterating the process until E satisfies a convergence condition.

In the algorithm used in Step SA3, the first virtual bone model created based on the image information of the bone surface that is obtained from a scan of the bone surface is roughly registered to the pre-stored second virtual bone model in order to make the two models as close as possible. After that, the RANSAC algorithm is used to remove possible error points, and the ICP algorithm is then run until the convergence condition is satisfied. Thus, a final fitted registration matrix is obtained.
Preferably, in Step SA4, individual coordinate systems, i.e., a coordinate system T1 of the navigation device 200, a coordinate system T2 of the fiducial marker, a coordinate system T3 of the stereoscopic vision scanner 100 and the navigation image coordinate system T4 of the pre-stored second virtual bone model are made consistent.
Step SA4 may include: determining a first coordinate transformation between the coordinate system T3 of the stereoscopic vision scanner and the coordinate system T2 of the fiducial marker based on a predetermined connection relationship between the stereoscopic vision scanner 100 and the fiducial marker 300; determining a second coordinate transformation between the coordinate system T2 of the fiducial marker and the coordinate system T1 of the navigation device based on the position of the fiducial marker 300 determined by the navigation device 200; determining a third coordinate transformation between the coordinate system T3 of the stereoscopic vision scanner and the navigation image coordinate system T4 based on the results of the registration of the first virtual bone model to the second virtual bone model in Step SA3; and deriving coordinates of the target object in the navigation image coordinate system T4 by performing a coordinate transformation on the first virtual bone model based on the first, second and third coordinate transformations.

Specifically, referring to Fig. 6, relative positions of the stereoscopic vision scanner 100 and the fiducial marker 300, and hence the first coordinate transformation between the coordinate system T3 of the stereoscopic vision scanner and the coordinate system T2 of the fiducial marker, can be derived from the predetermined connection relationship between the stereoscopic vision scanner 100 and the fiducial marker 300, e.g., from a mechanical design document or by calibration. Since the navigation device 200 and the fiducial marker 300 are adapted to each other so that the navigation device 200 is able to acquire positional information of the fiducial marker 300 in real time, the transformation between the coordinate system T2 of the fiducial marker and the coordinate system T1 of the navigation device can be obtained through tracking the fiducial marker 300 by the navigation device 200. From the results of the registration of the first virtual bone model to the second virtual bone model in Step SA3, the transformation between the coordinate system T1 of the navigation device and the navigation image coordinate system T4 can be obtained. Specifically, these transformations can be described by:

$$T_2 = M_3^2 * T_3$$

$$T_1 = M_2^1 * T_2$$

$$T_4 = M_1^4 * T_1$$

where $M_3^2$, $M_2^1$ and $M_1^4$ represent transformation matrices for the first, second and third coordinate transformations. The transformation matrix $M_3^2$ for the first coordinate transformation is used to transform the first virtual bone model created from the image information of the bone surface acquired by the stereoscopic vision scanner 100 from the coordinate system T3 of the stereoscopic vision scanner to the coordinate system T2 of the fiducial marker. The transformation matrix $M_2^1$ for the second coordinate transformation determined by tracking the fiducial marker 300 by the navigation device 200 is used to transform the image information of the bone surface from the coordinate system T2 of the fiducial marker to the coordinate system T1 of the navigation device. Additionally, the transformation between the coordinate system T1 of the navigation device and the navigation image coordinate system T4 is derived from the results of the registration of the first virtual bone model to the second virtual bone model in Step SA3. In this way, based on the tracking of the fiducial marker 300 by the navigation device 200, coordinates of the first virtual bone model created from the image information of the bone surface acquired by the stereoscopic vision scanner 100 in the navigation image coordinate system T4 can be obtained. That is, the registration of the bone site is achievable.

In this embodiment, when the stereoscopic vision scanner 100 is held by the operator, it may have a variable position relative to the patient. Since the registration matrix cannot work for navigation in a moving coordinate system, the coordinate system T1 of the navigation device is set as a world coordinate system, and the first virtual bone model is transformed to the coordinate system T1 of the navigation device. Additionally, the navigation device 200 is kept stationary once it has completed the scan. In this way, as long as the patient is also kept stationary, the navigation device 200 will have a fixed positional relationship relative to the patient. Once the navigation device 200 is kept stationary, the stereoscopic vision scanner 100 is allowed to be removed therefrom after the scan is completed, without affecting the subsequent steps.

It would be appreciated that the readable storage medium, the bone modeling registration system and the orthopedic surgical system provided in this embodiment are not limited to being used preoperatively, and if a need for registration emerges during surgery, for example, due to a leg movement of the patient, the stereoscopic vision scanner 100 can be still used to scan the bone at the new position for re-registration.

It is to be noted that as the bone surface is typically a layer of soft tissue, the image information of the bone surface captured by a direct scan of the stereoscopic vision scanner 100 generally contains point cloud data of the cartilage on the bone surface. Depending on a source of the second virtual bone model, two options may be provided for subsequent processing. In case of the pre-stored second virtual bone model being created based on image data from a nuclear magnetic resonance (MRI) scan, it will contain data of the cartilage on the bone. As the image information of the bone surface acquired from a scan of the stereoscopic vision scanner 100 also contain data of the cartilage, the above method can be directly used for registration.

If the pre-stored second virtual bone model is created from image data from a CT scan, it will not contain point cloud data of the cartilage tissue. In this case, the image information of the bone surface captured by the stereoscopic vision scanner 100 may be subjected to a compensation algorithm for extracting point cloud data of the bone surface. Accordingly, Step SA3 may include: obtaining cartilage compensation data using a cartilage compensation algorithm; correcting the first virtual bone model using the cartilage compensation data; and registering the corrected first virtual bone model to the second virtual bone model.

Optionally, in this embodiment, the cartilage may be ground and removed with a mill. As shown in Fig. 4, a target site 400 for cartilage removal is substantially in the form of a dot, and its location is determined by a surgeon during preoperative planning.

Fig. 5 demonstrates a scan performed after the cartilage is removed. After the removal, the stereoscopic vision scanner 100 may be used to scan the bone surface, and a single-site cartilage compensation algorithm may be run to derive the cartilage compensation data.

The single-site cartilage compensation algorithm may include the steps of: detecting an edge of the target site for cartilage removal of the target object using the Canny operator; calculating a gradient variation at the edge; fitting a depth variation in a region not for cartilage removal near the target site for cartilage removal based on the gradient variation; iterating these steps until the entire cartilage has been treated, thus deriving the cartilage compensation data for use in the subsequent registration process. It is to be noted that the Canny operator is a multi-stage edge detection algorithm. As the steps thereof including calculation of a gradient variation at an edge, fitting in a near region and iterations across the entire area are well known to those skilled in the art, further description thereof is omitted.

In summary, depending on the source of the pre-stored second virtual bone model, two different processing options are provided in this embodiment, without needing to place a fiducial marker on a bone. A preoperative image model can be registered to an intraoperative model of a physical bone, without causing secondary damage to a patient. Contact of surgical instruments with the patient's body and the chance of contamination are reduced, and cleaning of the instruments is facilitated. In addition, the bone modeling registration system is simple and allows easy deployment of the registration tool during surgery, a simplified registration process, lower surgical complexity and reduced surgical time. Further, compared with the traditional touch-based selection approach, the scanning approach employed by the stereoscopic vision scanner is more accurate, can better reflect the structure of a bone surface and enables higher registration accuracy.

## EMBODIMENT 2

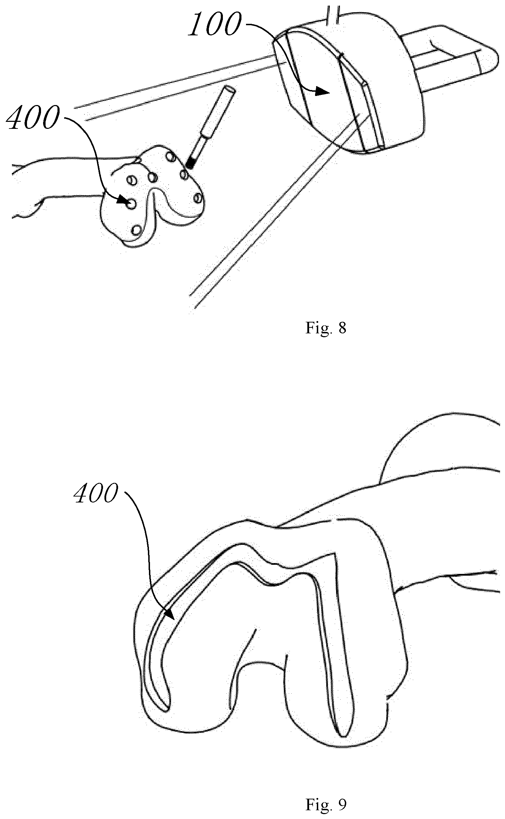

Reference is now made to Figs. 7 and 8. Fig. 7 schematically illustrates target sites for cartilage removal according to Embodiment 2 of the present invention. Fig. 8 schematically illustrates a scan performed after cartilage removal according to Embodiment 2.

A readable storage medium, a bone modeling registration system and an orthopedic surgical system are provided in Embodiment 2, which are substantially similar to the readable storage medium, the bone modeling registration system and the orthopedic surgical system of Embodiment 1. In the following, only features different from Embodiment 1 will be described, and those common to Embodiment 1 that have been described above will not be described again.

Embodiment 2 differs from Embodiment 1 in the derivation of cartilage compensation data for a second virtual bone model created from image data from a CT scan. Specifically, in Embodiment 2, cartilage may also be ground and removed with a mill. As shown in Fig. 7, the target sites for cartilage removal are substantially in the form of dots, and their locations may be determined by a surgeon during preoperative planning.

Fig. 8 demonstrates a scan performed after the cartilage is removed. The target sites may be treated in any desired order. After the removal, the stereoscopic vision scanner 100 may be used to directly scan the bone surface, and a multi-site cartilage compensation algorithm may be run to derive cartilage compensation data.

The multi-site cartilage compensation algorithm may include the steps of: detecting edges of the multiple target sites using the Canny operator; calculating gradient variations at the edges; based on the gradient variations, fitting surrounding regions of the target sites to derive depths of removal; and obtaining the cartilage compensation data through gradual extension of the fitted areas until the entire cartilage has been treated.

When any two of the fitted areas expend to interface with each other, a depth of removal at the interface may be determined by fitting based on a gradient variation at a center of the target site that is closer to the interface.

The multi-site cartilage compensation algorithm employed in this embodiment provides higher fitting accuracy than the single-site cartilage compensation algorithm used in the first embodiment. However, it requires cartilage removal at multiple target sites on the bone.

## EMBODIMENT 3

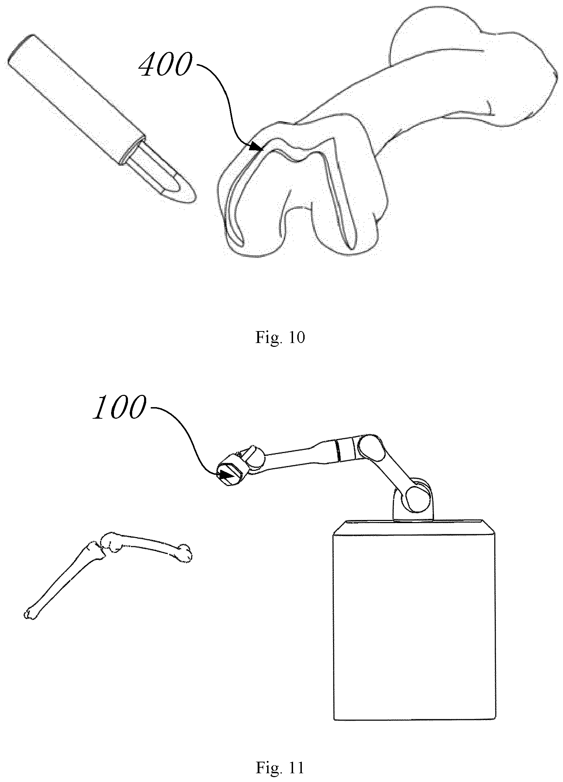

Reference is now made to Figs. 9 and 10. Fig. 9 schematically illustrates target sites for cartilage removal according to Embodiment 3 of the present invention. Fig. 10 schematically illustrates a scan performed after cartilage removal in Embodiment 3.

A readable storage medium, a bone modeling registration system and an orthopedic surgical system are provided in Embodiment 3, which are substantially similar to the readable storage medium, the bone modeling registration system and the orthopedic surgical system of Embodiment 1. In the following, only features different from the Embodiment 1 will be described, and those common to Embodiment 1 that have been described above will not be described again.

Embodiment 3 differs from Embodiment 1 in the derivation of cartilage compensation data for a second virtual bone model created from image data from a CT scan. Specifically, in Embodiment 3, cartilage may be removed by scribing with a scalpel. As shown in Fig. 9, the target sites for cartilage removal are substantially in the form of elongate areas, and their locations may be determined by a surgeon during preoperative planning.

Fig. 10 demonstrates a scan performed after the cartilage is removed. After the removal, the stereoscopic vision scanner 100 may be used to scan the bone surface, and a cartilage compensation algorithm for the scribed region may be run to derive the cartilage compensation data.

This cartilage compensation algorithm is substantially similar to the single-site compensation algorithm used in Embodiment 1. Specifically, a depth of removal for a surrounding region is fitted based on a gradient variation at an edge of the scribed region, and the cartilage compensation data is obtained by gradual extension of the fitted region until the entire cartilage has been treated. For more details in this regard, reference can be made to the foregoing description in Embodiment 1, and further description thereof is omitted.

## EMBODIMENT 4

Reference is now made to Fig. 11, a schematic illustration of a bone modeling registration system according to Embodiment 4 of the present invention.

A readable storage medium, the bone modeling registration system and an orthopedic surgical system are provided in Embodiment 4, which are substantially similar to the readable storage medium, the bone modeling registration system and the orthopedic surgical system of Embodiment 1. In the following, only features different from Embodiment 1 will be described, and those common to Embodiment 1 that have been described above will not be described again.

As shown in Fig. 11, differing from the bone modeling registration system in Embodiment 1, the bone modeling registration system in Embodiment 4 does not include a navigation device 200 or a fiducial marker 300. Instead, it includes a stereoscopic vision scanner 100 having a fixed position and posture, which can be determined by calibration. In an exemplary implementation, the stereoscopic vision scanner 100 is held by a robotic arm, which is kept stationary after the position and posture calibration of the stereoscopic vision scanner 100 in order to maintain the stereoscopic vision scanner 100 at the calibrated position and posture.

Since the position and posture of the stereoscopic vision scanner 100 are fixed, a coordinate transformation from a first virtual bone model created from image information of a bone surface that it captures to a world coordinate system is known. Therefore, once the first virtual bone model is registered to a second virtual bone model, a registration matrix for the first and second virtual bone models can be directly obtained.

Accordingly, a program stored in the readable storage medium, when executed, captures image information of a bone surface of a target object in real time from a stereoscopic vision scanner having a fixed position and posture, derives first virtual bone model in real time by 3D reconstruction from the real-time image information of the bone surface, registers the first virtual bone model in real time to a pre-stored second virtual bone model of the target object, and derives coordinates of the target object in a navigation image coordinate system in real time.

Further, after the registration matrix is obtained, the position and posture of the stereoscopic vision scanner 100 are maintained, and a continuous scan of the bone surface is conducted so that the registration algorithm is always run to update the registration matrix in real time, thereby enabling real-time tracking. With this arrangement, real-time registration is possible even when the bone experiences a change in position during surgery.

**EMBODIMENT 5**

A readable storage medium, a bone modeling registration system and an orthopedic surgical system are provided in Embodiment 5 of the present invention, which are substantially similar to the readable storage medium, the bone modeling registration system and the orthopedic surgical system of Embodiment 1. In the following, only features different from Embodiment 1 will be described, and those common to Embodiment 1 that have been described above will not be described again.

Embodiment 5 differs from Embodiment 1 in the derivation of cartilage compensation data for a second virtual bone model created from image data from a CT scan. Specifically, in Embodiment 5, instead of mechanically by a mill, a scalpel or the like, cartilage compensation can be achieved by automatically clearing cartilage data using a neural network trained with a machine learning algorithm. Specifically, this cartilage compensation algorithm may include deriving cartilage compensation data from a calculation performed on a first virtual bone model by the neural network that has been trained on a training set. It is noted that those skilled in the art would know how to train a neural network on a training set, and further description thereof is omitted herein.

It is to be noted that the readable storage media, the bone modeling registration systems and the orthopedic surgical systems in the above embodiments have been described in a progressive manner, and features of the embodiments can be combined without departing from the scope of the present invention.

The description presented above is merely that of a few preferred embodiments of the present invention and does not limit the scope thereof in any sense. Any and all changes and modifications made by those of ordinary skill in the art based on the above teachings fall within the scope as defined in the appended claims.

**Claims**

1. A readable storage medium, storing thereon a program, which, when executed,

   acquires image information of a bone surface of a target object from a stereoscopic vision scanner,
   creates a first virtual bone model by three-dimensional reconstruction according to the image information of the bone surface,
   registers the first virtual bone model to a pre-stored second virtual bone model of the target object, and
   obtains coordinates of the target object in a navigation image coordinate system based on position and posture information of the stereoscopic vision scanner.

2. The readable storage medium according to claim 1, wherein the image information of the bone surface comprises data of at least two images captured from different views.

3. The readable storage medium according to claim 1, wherein the creation of the first virtual bone model by three-dimensional reconstruction according to the image information of the bone surface comprises:

   obtaining point cloud data of a bone contour by segmenting the image information of the bone surface; and
   creating the first virtual bone model by three-dimensional reconstruction based on the point cloud data of the bone contour.

4. The readable storage medium according to claim 1, wherein the obtainment of the coordinates of the target object in the navigation image coordinate system based on the position and posture information of the stereoscopic vision scanner comprises:

   deriving a first coordinate transformation between a coordinate system of the stereoscopic vision scanner and a coordinate system of a fiducial marker based on a predetermined connection relationship of the stereoscopic vision scanner and the fiducial marker;
   deriving a second coordinate transformation between the coordinate system of the fiducial marker and a coordinate system of a navigation device based on a position of the fiducial marker determined by the navigation device;
   deriving a third coordinate transformation between the coordinate system of the stereoscopic vision scanner and the navigation image coordinate system based on a result of the registration of the first virtual bone model to the second virtual bone model; and

obtaining the coordinates of the target object in the navigation image coordinate system by performing a coordinate transformation based on the first, second and third coordinate transformations.

**5.** The readable storage medium according to claim 1, wherein the pre-stored second virtual bone model is created according to a magnetic resonance imaging scan of the bone surface of the target object.

**6.** The readable storage medium according to claim 1, wherein the pre-stored second virtual bone model is created based on a computerized tomography scan of the bone surface of the target object, and the registration of the first virtual bone model to the pre-stored second virtual bone model of the target object comprises:

deriving cartilage compensation data by a cartilage compensation algorithm; and

correcting the first virtual bone model based on the cartilage compensation data and registering the corrected first virtual bone model to the second virtual bone model.

**7.** The readable storage medium according to claim 6, wherein the cartilage compensation algorithm comprises: detecting an edge of a target site for cartilage removal of the target object using a Canny operator; calculating a gradient variation at the edge; fitting a depth variation in a region not for cartilage removal near the target site based on the gradient variation; deriving the cartilage compensation data by iteration until the cartilage has been entirely treated.

**8.** The readable storage medium according to claim 6, wherein the cartilage compensation algorithm comprises: detecting edges of a plurality of target sites for cartilage removal of the target object using a Canny operator; calculating gradient variations at the edges; based on the gradient variations, fitting surrounding regions of the target sites to derive depths of removal; and deriving the cartilage compensation data through gradual extension of the fitted regions until the cartilage has been entirely treated.

**9.** The readable storage medium according to claim 8, wherein when any two of the fitted regions expend to interface with each other, a depth of removal at the interface is determined by fitting based on a gradient variation at a center of the target site that is closer to the interface.

**10.** The readable storage medium according to claim 6, wherein the cartilage compensation algorithm comprises deriving the cartilage compensation data from a calculation performed on the first virtual bone model by a neural network that has been trained on a training set.

**11.** The readable storage medium according to claim 1, wherein the registration of the first virtual bone model to the second virtual bone model comprises:

roughly registering the first virtual bone model to the second virtual bone model;

removing possible error points using a random sample consensus algorithm; and

running an iterative closest point algorithm until a predefined convergence condition is satisfied, and obtaining a fitted registration matrix.

**12.** The readable storage medium according to claim 1, wherein the program, when executed,

acquires real-time image information of the bone surface of the target object from a stereoscopic vision scanner having a fixed position and posture,

creates a real-time first virtual bone model by three-dimensional reconstruction from the real-time image information of the bone surface,

registers the first virtual bone model to the pre-stored second virtual bone model of the target object in real time, and

obtains a real-time coordinate of the target object in the navigation image coordinate system.

**13.** A bone modeling registration system, comprising a processor and a stereoscopic vision scanner, the stereoscopic vision scanner configured to capture image information of a bone surface of a target object and feed the image information of the bone surface to the processor,

the processor communicatively connected to the stereoscopic vision scanner and configured to acquire position and posture information of the stereoscopic vision scanner and the image information of the bone surface of the target object captured by the stereoscopic vision scanner, create a first virtual bone model by three-dimensional reconstruction according to the image information of the bone surface, register the first virtual bone model to a pre-stored second virtual bone model, and obtain coordinates of the target object in a navigation image coordinate system based on the position and posture information of the stereoscopic vision scanner.

**14.** The bone modeling registration system according to claim 13, wherein the stereoscopic vision scanner has a fixed position and posture, which are determined by calibration.

**15.** The bone modeling registration system according to claim 13, further comprising a navigation device and a fiducial marker, the fiducial marker connected to the stereoscopic vision scanner, the navigation device adapted to the fiducial marker and configured to acquire real-time coordinate information of the fiducial marker and to transmit the real-time coordinate information to the processor, wherein the processor is communicatively connected to the navigation device and configured to obtain the position and posture information of the stereoscopic vision scanner based on the real-time coordinate information of the fiducial marker fed back from the navigation device.

16. An orthopedic surgical system, comprising the bone modeling registration system according to any one of claims 13 to 15 and at least one of a robotic arm, a surgical cart and a navigation cart.

Fig. 1

| | |
|---|---|
| Movement of Surgical Cart and Navigation Cart to Respective Proper Locations beside Hospital Bed | *SK1* |
| Deployment of Navigation Markers, Osteotomy Guide and Other Necessary Components | *SK2* |
| Preoperative Planning | *SK3* |
| Real-time Bone Registration | *SK4* |
| Deployment of Robotic Arm in Position for Surgical Operation | *SK5* |

Fig. 2

Fig. 3

400

Fig. 4

100

400

Fig. 5

Fig. 6

Fig. 7

Fig. 8

Fig. 9

400

Fig. 10

100

Fig. 11

# EP 4 223 246 A1

## INTERNATIONAL SEARCH REPORT

| | International application No. |
|---|---|
| | **PCT/CN2021/108603** |

**A. CLASSIFICATION OF SUBJECT MATTER**

A61B 34/20(2016.01)i; A61B 34/10(2016.01)i

According to International Patent Classification (IPC) or to both national classification and IPC

**B. FIELDS SEARCHED**

Minimum documentation searched (classification system followed by classification symbols)

A61B

Documentation searched other than minimum documentation to the extent that such documents are included in the fields searched

Electronic data base consulted during the international search (name of data base and, where practicable, search terms used)

CNTXT, WPABSC, ENTXTC, CNKI: 扫描, 骨, 表面, 配准, 注册, 无, 标记, 标志, 标靶, 导航, 软骨, 补偿; VEN, WPABS, ENTXT: scan, bone, surface, register, no, without, marker, marking, target, reference, navigate, gristle, cartilage, redress, recoup, compensate, compensation

**C. DOCUMENTS CONSIDERED TO BE RELEVANT**

| Category* | Citation of document, with indication, where appropriate, of the relevant passages | Relevant to claim No. |
|---|---|---|
| PX | CN 112155732 A (SUZHOU WEICHUANG CHANGXING ROBOTS CO., LTD.) 01 January 2021 (2021-01-01)<br>claims 1-16 | 1-16 |
| PX | CN 112155733 A (SUZHOU WEICHUANG CHANGXING ROBOTS CO., LTD.) 01 January 2021 (2021-01-01)<br>description, paragraphs 5-159, and figures 1-17 | 1-16 |
| PX | CN 112155734 A (SUZHOU WEICHUANG CHANGXING ROBOTS CO., LTD.) 01 January 2021 (2021-01-01)<br>description, paragraphs 5-157, and figures 1-17 | 1-16 |
| X | US 2013102893 A1 (VOLLMER FRITZ et al.) 25 April 2013 (2013-04-25)<br>description, paragraphs 5-46, and figures 1-5 | 1-16 |
| Y | CN 109785374 A (BEIHANG UNIVERSITY) 21 May 2019 (2019-05-21)<br>description, paragraphs 26-62, and figures 1-4 | 1-16 |
| Y | CN 106344152 A (SHENZHEN INSTITUTES OF ADVANCED TECHNOLOGY, CHINESE ACADEMY OF SCIENCES et al.) 25 January 2017 (2017-01-25)<br>description, paragraphs 100-210, and figures 1-4 | 1-16 |

☑ Further documents are listed in the continuation of Box C.    ☑ See patent family annex.

| \* | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **14 October 2021** | **27 October 2021** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/108603** |

C.     DOCUMENTS CONSIDERED TO BE RELEVANT

| Category* | Citation of document, with indication, where appropriate, of the relevant passages | Relevant to claim No. |
|---|---|---|
| A | CN 109692041 A (HANGZHOU JIANJIA ROBOTS CO., LTD.) 30 April 2019 (2019-04-30) entire document | 1-16 |

Form PCT/ISA/210 (second sheet) (January 2015)

**INTERNATIONAL SEARCH REPORT**
Information on patent family members

International application No.

**PCT/CN2021/108603**

| Patent document cited in search report | | | Publication date (day/month/year) | Patent family member(s) | | | Publication date (day/month/year) |
|---|---|---|---|---|---|---|---|
| CN | 112155732 | A | 01 January 2021 | None | | | |
| CN | 112155733 | A | 01 January 2021 | None | | | |
| CN | 112155734 | A | 01 January 2021 | None | | | |
| US | 2013102893 | A1 | 25 April 2013 | US | 9241657 | B2 | 26 January 2016 |
| | | | | EP | 2588018 | B1 | 07 December 2016 |
| | | | | WO | 2012000542 | A1 | 05 January 2012 |
| | | | | EP | 2588018 | A1 | 08 May 2013 |
| CN | 109785374 | A | 21 May 2019 | CN | 109785374 | B | 04 December 2020 |
| CN | 106344152 | A | 25 January 2017 | CN | 106344152 | B | 28 April 2020 |
| CN | 109692041 | A | 30 April 2019 | None | | | |

Form PCT/ISA/210 (patent family annex) (January 2015)